# EUROPEAN PATENT SPECIFICATION

(11) **EP 4 176 907 B1**
(45) Date of publication and mention of the grant of the patent: **16.07.2025**
(21) Application number: 20959839.0
(22) Date of filing: 30.10.2020
(51) Int. Cl.: A61L 27/36, A61L 27/60

(54) **SKIN GRAFT SUPPLEMENTING/CUSHIONING MEMBER FOR AURICLE SKIN DEFECT**
HAUTTRANSPLANTATERGÄNZUNGS-/DÄMPFUNGSELEMENT FÜR OHRHAUTDEFEKT
ÉLÉMENT DE COMPLÉMENTATION/D'AMORTISSEMENT DE GREFFE DE PEAU POUR DÉFAUT CUTANÉ DU PAVILLON DE L'OREILLE

(43) Date of publication of application: 10.05.2023
(73) Proprietor: National University Corporation University Of Toyama, Toyama-shi, Toyama 930-8555 (JP); Sakura Seiki Co., Ltd, Chikuma-shi, Nagano 387-0015 (JP)
(72) Inventor: FUJISAKA Michiro, Toyama-shi, Toyama 930-0194 (JP); SHOJAKU Hideo, Toyama-shi, Toyama 930-0194 (JP); ITO Shinsuke, Toyama-shi, Toyama 930-0194 (JP); YOSHIDA Toshiko, Toyama-shi, Toyama 930-0194 (JP); OKABE Motonori, Toyama-shi, Toyama 930-0194 (JP); SOKO Chika, Toyama-shi, Toyama 930-0194 (JP); ARAKAWA Masahiko, Chikuma-shi, Nagano 387-0015 (JP)
(74) Representative: Mewburn Ellis LLP
(86) International application number: PCT/JP2020/040753
(87) International publication number: WO 2022/091321

(56) References cited:
- JP-A- 2007 054 015
- SHOJAKU HIDEO ET AL: "Effect of hyperdry amniotic membrane patches attached over the bony surface of mastoid cavities in canal wall down tympanoplasty : Effect of Hyperdry Amniotic Membrane Patches", THE LARYNGOSCOPE, vol. 121, no. 9, 16 August 2011 (2011-08-16), United States, pages 1953 - 1957, XP093060047, ISSN: 0023-852X, Retrieved from the Internet <URL:https://api.wiley.com/onlinelibrary/tdm/v1/articles/10.1002%2Flary.22082> DOI: 10.1002/lary.22082
- FUJISAKA MICHIRO: "P009 Application to subepithelial tissue regeneration of HD human dried amnion", JOURNAL OF OTOLARYNGOLOGY OF JAPAN, vol. 121, no. 4, 1 January 2018 (2018-01-01), JP , pages 595, XP009537218, ISSN: 0030-6622
- OKABE MOTONORI, KITAGAWA KIYOTAKA, YOSHIDA TOSHIKO, SUZUKI TAKUMA, WAKI HIROKI, KOIKE CHIKA, FURUICHI ETSUKO, KATOU KIYOSHI, NOMUR: "Hyperdry human amniotic membrane is useful material for tissue engineering: Physical, morphological properties, and safety as the new biological material", JOURNAL OF BIOMEDICAL MATERIALS RESEARCH PART A, vol. 102, no. 3, 1 March 2014 (2014-03-01), US , pages 862 - 870, XP055938671, ISSN: 1549-3296, DOI: 10.1002/jbm.a.34753
- KANEKO TSUYOSHI: "Microtia auricular reconstruction and potential use of tissue engineering", JOURNAL OF CLINICAL AND EXPERIMENTAL MEDICINE, vol. 229, no. 9, 30 November 2008 (2008-11-30), JP , pages 876 - 880, XP009537274, ISSN: 0039-2359

## Description

### Technical Field

The present invention relates to a filling/cushioning member for use in a method of treating auricular skin defects.

### Background Art

It is known that when skin grafting is performed on a defect following skin removal treatment due to the onset of cancer or the like, there are parts of the body where engraftment is difficult. For auricular cancer (basal cell carcinoma), even when skin grafting is performed on an auricular skin defect, infection is likely and engraftment failure is common.

Amniotic membrane has come into use as an effective dressing for intractable wounds.

### Citation List

### Patent Literature

Patent Literature 1: Japanese Laid-open Patent Publication No. 2007-54015

### Non Patent Literature

Non Patent Literature 1: Gruss, J. S. & Jirsch, D. W. Human amniotic membrane: A versatile wound dressing. Can. Med. Assoc. J. 118, 1237-1246 (1978).
Non Patent Literature 2:Okabe, M. et al. Hyperdry human amniotic membrane is useful material for tissue engineering: Physical, morphological properties, and safety as the new biological material. J. Biomed. Mater. Res. - Part A 102, 862-870 (2014).

### Summary of Invention

### Technical Problem

Amniotic membrane is a tough biological membrane composed of collagen and elastic fibers, and raw amniotic membrane has been reported as being a useful covering material for trauma and burns (see Non Patent Literature 1).

However, raw amniotic membrane has not been available immediately when needed and has been complicated to store and handle, which has limited its use in actual clinical practice. On the other hand, dry amniotic membrane (or "hyper-dry amniotic membrane": hereinafter, "HD-AM") produced by a specific drying process has also been announced (see Patent Literature 1 and Non Patent Literature 2).

Although skin grafting is performed on defects produced by treatment that removes auricular skin at the onset of cancer or the like, engraftment can be difficult depending on the location. For auricular cancer (basal cell carcinoma) in particular, even when skin grafting is performed on an auricular skin defect, infection is likely and engraftment failure is common.

Negative pressure wound therapy (NPWT) on defects to the perichondrium or subcutaneous tissue is a wound care method in which a foam material, such as polyurethane, is placed on a wound, the entire wound is covered with a film material, and negative pressure is continuously applied to the covered wound to promote wound healing.

As an advantage of the present method, the research conducted here found that a favorable scaffold could be formed by placing hyper-dry amniotic membrane on cartilage that has become exposed due to a defect in the perichondrium and/or subcutaneous tissue.

Conventional skin grafting was poor and has not had a material with an effect of directly promoting healing. The sole benefit was avoiding necrosis of the affected area.

### Solution to Problem

The present inventors established that HD-AM functions as a scaffold for cells and promotes tissue regeneration, which contributes to engraftment after a skin graft, thereby completing the present invention.

A filling/cushioning member according to the present invention is a restorative member for use in a method of treating auricular skin defects and comprises a dry amniotic membrane produced by performing a drying process on raw amniotic membrane that surrounds a fetus of an animal, including a human, wherein the dry amniotic membrane has been dehydrated and dried so as to enable storage in an aseptic dry atmosphere, and amniotic membrane produced by rehydrating the dry amniotic membrane by immersion in water or a buffer retains epithelial cells, basement membrane, and connective tissue constituting the raw amniotic membrane.

By using this configuration, the active effect of promoting wound healing promotes engraftment of a skin graft when the filling / cushioning member is placed as a scaffold between a skin graft and a perichondrium defect or a subcutaneous tissue defect.

### Advantageous Effects of Invention

By using the filling/cushioning member according to the present invention during a skin grafting operation on a perichondrium defect or subcutaneous tissue defect, it is possible to promote engraftment of a skin graft through the active effect of amniotic membrane in promoting wound healing.

### Brief Description of Drawings

FIG. 1 is a diagram useful in explaining a first skin graft following excision of auricular cancer (basal cell carcinoma). Excision was performed, and skin grafting was performed on the resulting auricular skin defect. Pathological results from a month and a half later found residual cancer, exposed cartilage, infected necrotic areas, pus discharge, and no engraftment of the skin graft.
FIG. 2 is a diagram explaining excision of recurrent basal cell carcinoma. 1. With a safety margin, the tissue was collected around the circumference and submitted for rapid pathological diagnosis. The absence of cancer cells was confirmed. 2. While observing with an endoscope, a circumferential incision was made in the skin of the external auditory canal at a position just before the eardrum. A resection stump was created on the external auditory canal side.
FIG. 3 is a diagram explaining a skin graft to which HD-AM has been applied. In FIG. 3A, the HD-AM was applied over the external auditory canal and the auricular cartilage. In FIG. 3B, a skin graft was performed on top of the HD-AM.
FIG. 4 is a diagram useful in explaining treatment results of skin grafting with HD-AM. Two months later, the skin graft had taken root and was in good condition.
FIG. 5 depicts drying equipment for preparing HD-AM.

### Description of Embodiments

### Overview of Embodiments

A dried amniotic membrane manufactured by a specified drying process (called "hyperdrying") is, for example, the dried amniotic membrane described in Patent Literature 1. That is, raw amniotic membrane placed in a processing tank is continuously heated by a far-infrared heater provided inside the processing tank, and a depressurization operation, in which the inside of the processing tank is placed in a depressurized state, and irradiation of the raw amniotic membrane with microwaves from a microwave generating device provided inside the processing tank to apply energy to water molecules present inside the amniotic membrane and cause drying during a pressure recovery operation that slightly raises the pressure inside the depressurized processing tank toward atmospheric pressure were performed. In dried amniotic membrane (HD-AM) manufactured by repeating the above process a plurality of times, the amniotic membrane cells themselves are inactivated but the cell and tissue structures are retained.

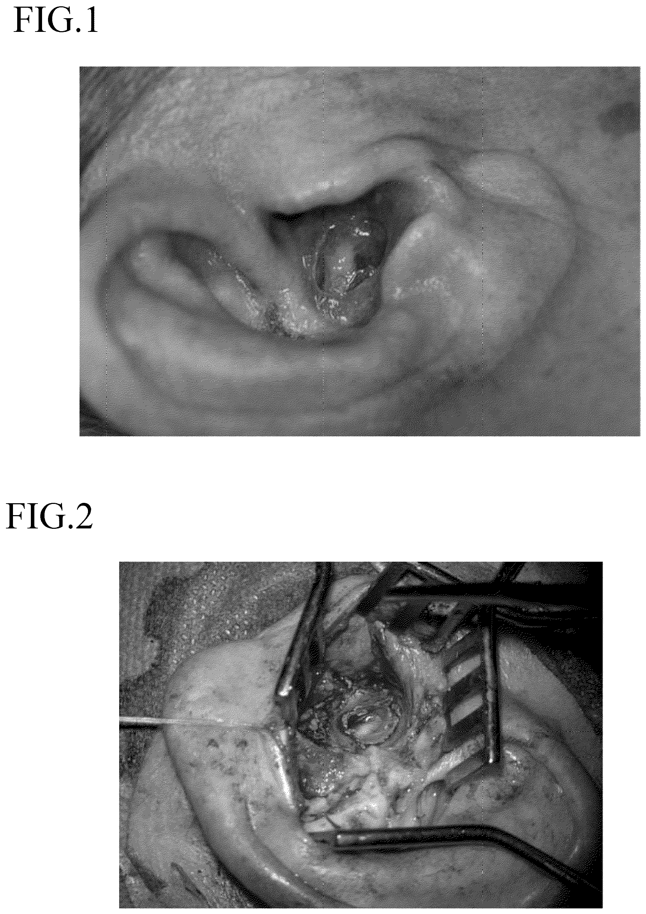

Excision of auricular cancer (basal cell carcinoma) was performed at another department, and then skin grafting was performed on the auricular skin defect. However, residual cancer was found at the wound, partial infection occurred, and engraftment failed (see FIG. 1).

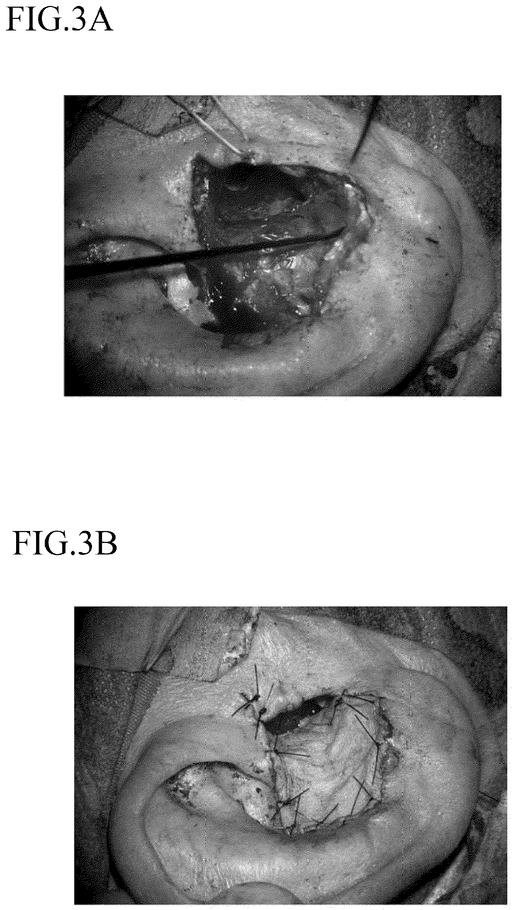

A safety margin was provided from the previously excised site, and after confirming through rapid pathological diagnosis that no cancer remained, the cancer was removed as a mass (see FIG. 2). HD-AM was applied to the surface of the exposed auricular cartilage (see FIG. 3A), and then harvested skin was grafted on top (see FIG. 3B).

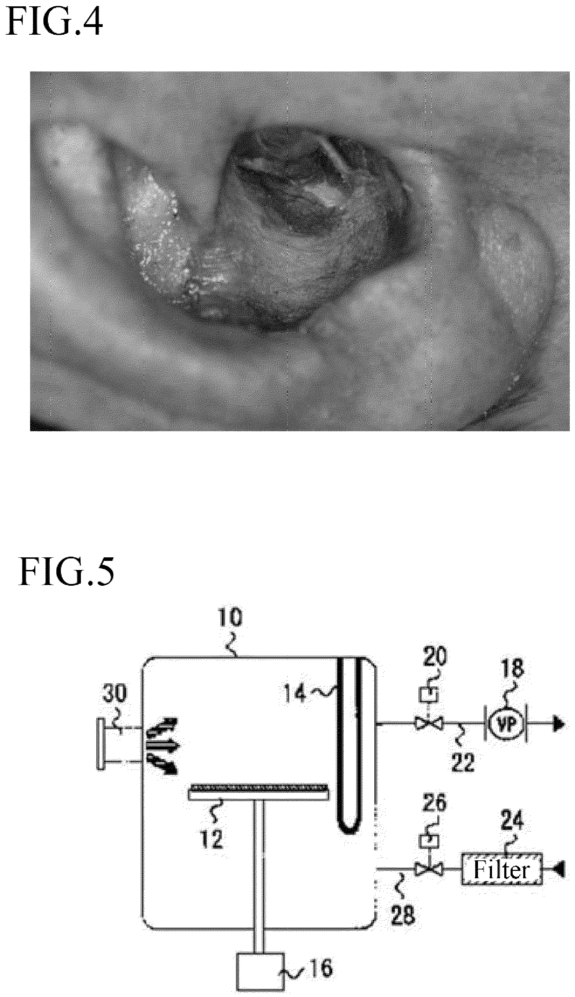

Two months later, the postoperative course was favorable (FIG. 4).

In the present embodiment, HD-AM is placed as a filling/cushioning member on a perichondrium defect or a subcutaneous tissue defect. By placing the HD-AM as a scaffold between a skin graft and a perichondrium defect or a subcutaneous tissue defect, the active effects of the amniotic membrane, such as the promotion of wound healing and formation of a scaffold, are promoted.

### Manufacture of HD-AM

Using the drying device depicted in FIG. 5, a raw amniotic membrane was dried with vacuum, far-infrared, and microwave devices set at the conditions given below.

The drying device in FIG. 5 is equipped, inside a processing tank 10, with a rotary table 12 on which raw amniotic membrane is placed, and a far-infrared heater 14 as a heating means. Also, to reduce the pressure inside the processing tank 10, a solenoid valve 20 and a vacuum pump 18 are connected to the processing tank 10. Also, to repressurize the processing tank 10 interior, a solenoid valve 26 and a filter 24 are connected to the processing tank 10.

The processing tank 10 is also provided with a microwave irradiation device 30 which performs, when a pressure recovery operation has been performed from a reduced pressure state, drying while applying energy to water molecules in the amniotic membrane.

Drying tank heating temperature: 50°C, FIR: 50°C, Stop valve: 37%, Maximum ultimate pressure 0.34kPa, Dry running maximum ultimate pressure 0.33kPa

### Drying method

(1) Depressurization 180 sec
(2) Pressure recovery 30 sec (with stop valve 37% open)
   Commence emission of microwaves
   0.1kw for 180 sec (pressure recovery continues)
(3) Depressurization 180 sec
(4) Repeat (2) and (3) thereafter
(5) End drying manually by checking the ultimate pressure after 180 sec of depressurization in (3) is (0.30 to 0.35 kPa).

Process complete when atmospheric pressure is restored

### Industrial Applicability

Dried amniotic membrane (HD-AM) is manufactured by a specific drying process, that is, raw amniotic membrane that has been placed inside a processing tank is continuously heated by a far-infrared heater provided inside the processing tank while performing a depressurization operation that reduces the pressure inside the processing tank and irradiation of the raw amniotic membrane with microwaves from a microwave generating device provided inside the processing tank to apply energy to the water molecules present inside the amniotic membrane and dry the amniotic membrane during a pressure recovery operation that slightly raises the pressure inside the depressurized processing tank toward atmospheric pressure. By repeating the above multiple times, it is possible to improve storage stability and handling of the dried amniotic membrane while preserving the cell and tissue structures. By using this HD-AM as a scaffold to prevent poor skin grafting on perichondrium/periosteal defects and subcutaneous tissue defects, it is possible to enhance the healing effect of skin grafts.

## Claims

1. A filling/cushioning member for perichondrium and subcutaneous tissue that is a restorative member for use in a method of treating auricular skin defects, wherein the filling / cushioning member is placed as a scaffold between a skin graft and a perichondrium defect or a subcutaneous tissue defect,
the filling/cushioning member comprising a dry amniotic membrane produced by performing a drying process on raw amniotic membrane that surrounds a fetus of an animal, including a human,
wherein the dry amniotic membrane has been dehydrated and dried so as to enable storage in an aseptic dry atmosphere, and
amniotic membrane produced by rehydrating the dry amniotic membrane by immersion in water or a buffer retains epithelial cells, basement membrane, and connective tissue constituting the raw amniotic membrane.

## Patentansprüche

1. Füll-/Polsterungselement für Perichondrium und subkutanes Gewebe, bei dem es sich um ein verstärkendes Element handelt, zur Verwendung in einem Verfahren zur Behandlung von Hautdefekten der Ohrmuschel, wobei das Füll-/Polsterungselement als Gerüst zwischen einem Hauttransplantat und einem Perichondrium-Defekt oder einem Defekt des subkutanen Gewebes platziert wird,
wobei das Füll-/Polsterungselement eine trockene Amnionmembran umfasst, die durch Ausführen eines Trocknungsvorgangs an einer rohen Amnionmembran hergestellt wird, die einen Fötus eines Tiers, einschließlich eines Menschen, umgibt,
wobei die trockene Amnionmembran dehydratisiert und getrocknet wurde, um eine Lagerung in einer aseptischen trockenen Atmosphäre zu ermöglichen, und
die Amnionmembran, die durch Rehydratisieren der trockenen Amnionmembran durch Eintauchen in Wasser oder einen Puffer hergestellt wird, Epithelzellen, die Basalmembran und Bindegewebe, welche die rohe Amnionmembran darstellen, beibehält.

## Revendications

1. Élément de remplissage/d'amortissement pour le périchondre et du tissu sous-cutané qui est un élément de restauration à utiliser dans un procédé de traitement de défauts cutanés auriculaires, dans lequel l'élément de remplissage/d'amortissement est placé sous la forme d'une échafaudage entre une greffe de peau et un défaut du périchondre ou un défaut de tissu sous-cutané,
l'élément de remplissage/d'amortissement comprenant une membrane amniotique sèche produite en effectuant un processus de séchage sur une membrane amniotique brute qui entoure un fœtus d'un animal, y compris un être humain,
dans lequel la membrane amniotique sèche a été déshydratée et séchée de manière à permettre un stockage dans une atmosphère sèche aseptisée, et
la membrane amniotique produite par réhydratation de la membrane amniotique sèche par immersion dans de l'eau ou un tampon retient les cellules épithéliales, la membrane basale et le tissu conjonctif constituant la membrane amniotique brute.
